# EUROPEAN PATENT SPECIFICATION

(11) **EP 1 409 705 B1**
(45) Date of publication and mention of the grant of the patent: **24.05.2006**
(21) Application number: 01928191.4
(22) Date of filing: 19.03.2001
(51) Int. Cl.: C12P 7/26

(54) **A PROCESS FOR THE PRODUCTION OF AN INHIBITOR OF HUMAN PLATELET AGGREGATION AND SOYBEAN LIPOXYGENASE**
VERFAHREN ZUR HERSTELLUNG VON EINEM INHIBITOR GEGEN HUMANE PLÄTTCHENAGGREGATION UND LIPOXYGENASE AUS SOJABOHNE
PROCEDE DE PRODUCTION D'UN INHIBITEUR DE L'AGREGATION PLAQUETTAIRE HUMAINE ET LIPOXYGENASE DU SOJA

(43) Date of publication of application: 21.04.2004
(73) Proprietor: Council of Scientific and Industrial Research;an Indian Reg. body incorporated under Reg. of Societies Act (Act XXI of 1860), New Delhi 110 001 (IN)
(72) Inventor: SATTUR, Avinash, Prahlad, Mysore Karnataka 570 013 (IN); RAO, Kadivala, Chandra, S., Mysore Karnataka 570 013 (IN); DIVAKAR, Soundar, Mysore Karnataka 570 013 (IN); KARANTH, Naikanakatte, Ganesh, Mysore Karnataka 570 013 (IN); SUNEETHA, Wesley, Jessie, Mysore Karnataka 570 013 (IN); KRISHNAKANTHA, Thirumalai, Parthasarathy, Mysore Karnataka 570 013 (IN); VISHWANATHA, Suryanarayana, Mysore Karnataka 570 013 (IN)
(74) Representative: Frith, Richard William
(86) International application number: PCT/IN2001/000043
(87) International publication number: WO 2002/074970

(56) References cited:
- US-A- 5 208 364
- JEFFERSON, WILLIAM E., JR.: "Steroid and other factors influencing the accumulation of asperenone and fermentation acids by Aspergillus niger in replacement cultures" BIOCHEMISTRY (1967), 6(11), 3484-8 , XP002183554
- JEFFERSON, WILLIAM E., JR.: "Isolation of characterization of asperenone, a new phenylpolyene from Aspergillus niger" BIOCHEMISTRY (1967), 6(11), 3479-84 , XP002183555
- J. YU ET AL.: "Asperyellone, a new yellow pigment of Aspergillus awamori and Aspergillus niger" AGRICULTURAL AND BIOLOGICAL CHEMISTRY, vol. 31, no. 7, 1967, pages 831-836, XP001041476 TOKYO JP
- PATTENDEN G: "SYNTHESIS OF ASPERENONE A NEW PIGMENT FROM ASPERGILLUS-NIGER AND ASPERGILLUS-AWAMORI" TETRAHEDRON LETTERS, vol. 46., 1969, pages 4049-4052, XP002183556 ISSN: 0040-4039
- PATTENDEN G: "SYNTHESIS OF ASPERENONE ALL-TRANS E-8 METHYL-13-PHENYLTRIDECA-4 6 8 10 12 PENTAEN-3-ONE A PIGMENT OF ASPERGILLUS SPECIES OF FUNGI" JOURNAL OF THE CHEMICAL SOCIETY SECTION C ORGANIC CHEMISTRY, no. 10, 1970, pages 1404-1409, XP002183557
- RABACHE M ET AL: "PHENYL PROPYLENES OF ASPERGILLUS-NIGER STRUCTURE AND PROPERTIES OF ASPERRUBROL" PHYTOCHEMISTRY (OXFORD), vol. 13, no. 3, 1974, pages 637-642, XP001041475 ISSN: 0031-9422
- AYER WILLIAM A ET AL: "Phenolic and other metabolites of Phellinus pini, a fungus pathogenic to pine." PHYTOCHEMISTRY (OXFORD), vol. 42, no. 5, 1996, pages 1321-1324, XP001041467 ISSN: 0031-9422
- PATENT ABSTRACTS OF JAPAN vol. 0, no. 0 (C-0) -& JP 06 256275 A (F. HOFFMANN LA ROCHE AG), 13 September 1994 (1994-09-13)
- PATENT ABSTRACTS OF JAPAN vol. 016, no. 455 (C-0987), 22 September 1992 (1992-09-22) -& JP 04 159274 A (TAISHO PHARMACEUT CO LTD), 2 June 1992 (1992-06-02) cited in the application
- BIOTECHNOLOGY LETTERS, vol. 24, no. 12, December 2002 (2002-12), pages 1967-1970, KLUWER ACADEMIC PUBLISHERS, THE NETHERLANDS

## Description

### Technical Field

The present invention relates to a process for the production of 8-methyl 13-phenyl trideca 4,6,8,10,12 pentene-3-one useful as inhibitors of human platelet aggregation as well as soybean lipoxygenase. The said compound having formula 1 is obtained from *Aspergillus niger.*

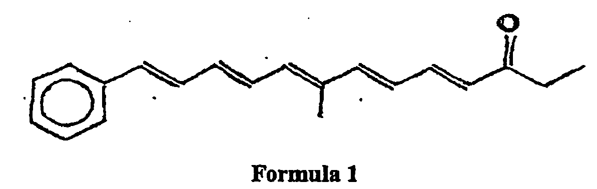

### Background Art

Japan Patent No.06256275 claimed a process for the production of a rabbit platelet aggregation inhibitor from *streptomyces sps.* However, a large number of medium constituents have been used for the production of the inhibitor.

Japan Patent No.04159274 claimed a benzoquinone derivative through fermentation as a rabbit platelet aggregation inhibitor. The activity claimed in the prior art is very low i.e. activity with an IC₅₀ of 1 mm.

United States Patent No.5208364 claimed a 5-lipoxygenase inhibitor from *Streptomyces sp.* The prior art describes an unwieldy process for using HPLC for purifying the inhibitor from the broth.

The preparation of lipoxygenase inhibitors is disclosed in Betina, V; Prog Ind Microbiol 30 (1994) 121-135. The prior art describes the production of these inhibitors, such as 3-methoxytropolone and kf 8940, from *streptoverticillium* sps and *pseudomonas methanica,* respectively. However, these metabolites have been reported to be produced from complex medium components.

Jefferson William E, Biochemistry (1967), 6 (11), p 3479 to 84 relates to a process for obtaining as perenone from Aspergillus niger employing an aqueous glycerol medium.

Studies on sulphasalazine metabolites, such as N-acetylaminosalicylic acid and 5-amino salicylic acids showing effective soybean lipoxygenase inhibition is disclosed in Allgayer,H; Eisenburg,J; Paumgartner,G. Eur J Clin Pharmacol. 26 (984) 449-451. However, this prior art has no disclosure or suggestion that soybean lipoxygenase inhibitors act as inhibitors against platelet aggregation as well.

From the above discussion describing the state of the art it is clear that there were no known or apparently simple fermentation medium and conditions to obtain molecules with activity against human platelet aggregation and acting as soybean lipoxygenase inhibitors.

Further, none of the above prior art disclosed preparation of the compound 8-methyl 13-phenyl trideca 4,6,8,10,12 pentene-3-one Formula 1 useful as a human platelet aggregation inhibitor and soybean lipoxygenase inhibitor.

### Summary of the invention

The objective of the present invention is to develop a process for the production of 8-methyl 13-phenyl trideca 4,6,8,10,12 pentene-3-one and exploit its use as human platelet aggregation inhibitor and soybean lipoxygenase inhibitor. The said compound has formula 1 as shown herein below.

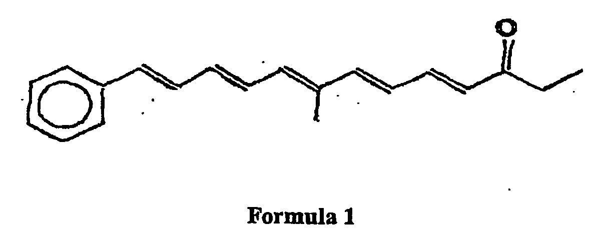

### Detailed description of the invention

Accordingly, the invention provides a process for the production of 8-methyl 13-phenyl trideca 4,6,8,10,12 pentene-3-one having formula 1 and obtained from *Aspergillus niger.* The said compound is useful as an inhibitor human platelet aggregation as well as soyabean lipoxygenase.

The process for the production of 8-methyl 13-phenyl trideca 4,6,8,10,12 pentene-3-one, comprises the steps of:
i) developing a medium comprising potato dextrose broth and having pH of 5-5.5,
ii) inoculating the medium in step (i) with fungi of the Aspergillus species and incubating the culture at 28° to 37°C for 5 to 8 days.
iii) adding ethyl acetate to the culture and incubating it at room temperature for 1-3 hrs.,
iv) separating the solvent from the fermentation broth by filtration,
v) removing all traces of water from the culture, and drying it to obtain a crude extract,
vi) subjecting the crude extract to column chromatography and eluting the compound 8-methyl 13-phenyl trideca 4,6,8,10,12 pentene-3-one as red crystals from the orange colored fraction, and
vii) purifying the compound by conventional methods.

To describe in detail, the process comprises the steps of developing an inoculum for fermentation by scraping the mycelia or spores of *aspergillus niger* from a 3-5 day old potato dextrose agar slant transferring to a 500-2000 ml potato dextrose broth solution and incubating to 28-37°C for 24-48 hours under agitation at 100-200 rpm with the fermentation being carried out in a fermentor of 10-15 litres capacity, wherein the medium used for both inoculum development and main fermentation is potato dextrose. The broth comprises boiled 15-20 g of peeled and cut potatoes and 1-3 g of glucose for 100 ml of the medium, with final pH being 5.1-5.5 and the medium being sterilized at 121°C for 10-20 minutes and allowed to cool to 30°C before inoculation. Thereafter, 0.5-2 litres of inoculum is transferred to the liquid medium and incubated at 28-30°C for 5-8 days at 100-200 rpm. At the end of the fermentation process, 1-5 litres of solvent such as ethylacetate is added to the fermented medium and incubated at room temperature for 1-3 hours, after which the fermented broth is filtered through whatman no 1 filter paper and the solvent is separated using a separating funnel. The next step is to add 10-50 g of anhydrous sodium sulphate to remove any traces of residual water. The solvent is distilled under vacuum to give a crude extract of 0.5-2g, which is loaded on 10-50g of silica gel (100-200 mesh) column in hexane, wherein the column has a bed volume of 20-60 ml. After which the column is first washed with 1-2 volumes of hexane after this the inhibitor is eluted using 1-2 volumes of hexane:chloroform (1:1) in the form of an orange coloured fraction, which is distilled under vacuum to remove the solvent and obtain 100-200 mgs of this semi purified inhibitor, to which 1-10 ml of hexane is added and stored at 1-10°C for at least 2-3 days to obtain 1-5 mgs of the purified said inhibitor in the form of red crystals which has a final ic₅₀ activity of 234 µM against human platelet aggregation and an ic₅₀ activity of 95.6 µM against soybean lipoxygenase.

The aspergillus species may be selected from known species such as *A. flavus, A. japonicus, A. niger, A. awamori.* These strains are equivalent to strains such as ATCC 9029, ATCC 11414, ATCC 1015, ATCC 10522, ATCC 26550, NRRL 3, NRRL 556, NRRL 337, NRRL 1042, CBS 102,49, CMI 41876.

In another embodiment of the present invention, the liquid medium used is potato dextrose broth consisting of 15-20g of potato and 1-3 g of glucose for 100 ml of the medium.

In a further embodiment of the present invention, cultivation may be effected for 3-5 days and the age of the inoculum may be 24-48 hours grown under agitation at 100-200 rpm.

In yet another embodiment of the present invention, the liquid medium employed for fermentation may be sterilized at 121°C for 10-20 minutes and inoculated with 5 to 15% inoculum.

In still another embodiment of the present invention, the fermentation time for the production of the human platelet aggregation inhibitor may be between 5 to 8 days.

In still another embodiment of the present invention the fermented liquid medium may be extracted with water immiscible solvents like chloroform, ethylacetate.

In still another embodiment of the present invention the purification of the human platelet aggregation inhibitor comprises recovering the said inhibitor by conventional column chromatography using combinations of polar and non polar solvents.

The invention, thus, provides 8-methyl 13-phenyl trideca 4,6,8,10,12 pentene-3-one useful as human platelet aggregation inhibitor and inhibitor of soyabean lioxygenase activity.

The invention is illustrated in the following example.

### EXAMPLE

In a 15 litres capacity fermentor, 10 litres of fresh potato dextrose broth was prepared by boiling 200 g of peeled and sliced potatoes for 20 minutes. After cooling to room temperature, 200 g of dextrose was added, the pH was adjusted to 5.1. The flask was then autoclaved at 121°C for 15 minutes. To this 11 of a 24 hour old fungal inoculum was added and then incubated for 7 days at 30°C. At the end of the fermentation period, 4 litres of ethylacetate was added and the culture was incubated at room temperature for two hours. The solvent was filtered through whatman no.1 filter paper. Subsequently, the solvent was separated from the broth using a separating funnel and 50 g of anhydrous sodium sulphate was added to the solvent to remove any traces of residual water. The solvent was evaporated under vacuum to obtain 1.5g crude extract with platelet aggregation inhibition activity of 22.5%. This was loaded on 50g of silica gel (100-200 mesh) column in hexane to give bed volume of 40 ml. After washing the column with 100 ml of hexane, the inhibitor was eluted using 600 ml of hexane:chloroform (1:1). The orange colored fraction containing the semi purified said inhibitor was distilled under vacuum to remove the solvent. To 150 mgs of this semi purified inhibitor, 2 ml of hexane was added and stored in cold overnight to obtain 3.3 mgs of the purified inhibitor in the form of red crystals. 234 µM of the inhibitor gave 50% inhibition against human platelet aggregation and 95.6 µM gave 50% inhibition against soybean lipoxygenase.

Analytical data for the compound: 8-methyl 13-phenyl trideca 4,6,8,10,12 pentene-3-one
Molecular Formula: C₂₀H₂₂ O
Molecular Weight: 278
Color and physical appearance of inhibitor: red crystals
Solubility: highly soluble in chloroform, dimethylsulfoxide, acetonitrile
Absorption maxima (nm) at 30 µg/ml dmso: 255, 304 and 406 nm
¹H NMR spectrum (cdcl₃, δₜₘₛ = 0 ppm): 71.4 2h d 9.03hz; 7.31 2h t 9.00hz; 7.25 3h m 9.03 hz; 6.92 1h dd 16.1hz; 6.67 2h dd 18.1hz; 6.96 1h dd 16.1hz; 6.39 2h dd 16.1hz; 6.21 1h d 18.1hz; 1.14 3h 5 7.6hz; 1.97 3h s; 2.6 2h q 7.6hz.
Mass spectrum: m⁺ - ch₃ = 263; m⁺ - ch₃⁻ch₂ = 249; m⁺ - ch₃⁻ch₂-co + = 221; m⁺ - 91 = 187; 187-26 = 161; t 91+ ch=105
Activity of purified inhibitor; IC₅₀ of the inhibitor against platelet aggregation:234 µM
IC₅₀ of the inhibitor against soybean lipoxygenase (pH 9) is :95.6µM.

## Claims

1. A process for the production of 8-methyl 13-phenyl, trideca 4,6,8,10,12 pentene-3-one, useful as platelet aggregation inhibitor and soybean lipoxygenase inhibitor, said process comprising the steps of:
i) developing a medium comprising potato dextrose broth and having pH of 5-5.5,
ii) inoculating the medium in step (i) with fungi of the Aspergillus species and incubating the culture at 28° to 37° for 5 to 8 days,
iii) adding ethyl acetate to the culture and incubating it at room temperature for 1-3 hrs.,
iv) separating the solvent from the fermentation broth by filtration,
v) removing all traces of water from the culture, and drying it to obtain a crude extract,
vi) subjecting the crude extract to column chromatography and eluting the compound 8-methyl 13-phenyl trideca 4,6,8,10,12 pentene-3-one as red crystals from the orange colored fraction, and
vii) purifying the compound by conventional methods.

2. A process for the production of 8-methyl 13-phenyl trideca 4,6,8,10,12 pentene-3-one, useful as a human platelet aggregation inhibitor and soybean lipoxygenase inhibitor, which comprises developing inoculum for the fermentation by scraping the mycelia or spores of *Aspergillus* from a 3-5 day old potato dextrose agar slant and transferred to a 500-2000 ml potato dextrose broth solution and incubated at 28-37° for 24-48 hours under agitation at 100-200 rpm and thereby fermenting the broth, wherein the medium used for both inoculum development and main fermentation is potato dextrose broth comprising boiled 15-20 g of peeled and cut potatoes and 1-3 g of glucose for 100 ml of the medium, with the final pH being 5.1-5.5 and the medium being sterilized at 121°C for 10-20 minutes and allowed to cool to 30°C before inoculation, into which 0.5-2 litres of inoculum is transferred to the liquid medium and incubated at 28-30°C for 5-8 days at 100-200 rpm, and at the end of the fermentation 1-5 litres of solvent such as ethylacetate is added to the fermented medium and incubated at room temperature for 1-3 hours, after which the fermented broth is filtered through Whatman no. 1 filter paper and the solvent is separated using a separating funnel, adding 10-50 g of anhydrous sodium sulphate to remove any traces of residual water, and the solvent being distilled under vacuum to give a crude extract of 0.5-2 g which is purified by column chromatography employing a combination of polar and non-polar solvents wherein the crude extract is loaded on 10-50 g of silica gel (100-200 mesh) column in hexane, wherein the column has a bed volume of 20-60 ml, after which the column is first washed with 1-2 volumes of hexane after which the inhibitor is eluted using 1-2 volumes of hexane:chloroform (1:1) in the form of an orange coloured fraction, which is distilled under vacuum to remove the solvent and obtain 100-200 mgs of this semi purified inhibitor, to which 1-10 ml of hexane is added and stored at 1-10°C for at least 2-3 days to obtain 1-5 mgs of the purified said inhibitor in the form of red crystals which has a final ic₅₀ activity of 234 µM and 95.6 µM against human platelet aggregation and lipoxygenase inhibition, respectively.

3. A process as claimed in claim 2 wherein the fungi are selected from *Aspergillus flavus, Aspergillus japonicus, Aspergillus niger* and *Aspergillus awamori.*

4. A process as claimed in claim 2 wherein the medium in step (i) comprises potato and glucose.

5. A process as claimed in claim 2 wherein the Aspergillus are cultivated for 3-5 days in a medium containing potato, dextrose and agar under agitation at 100-200 rpm.

6. A process as claimed in claim 2 wherein the fermentation time for the production of the human platelet aggregation inhibitor and soybean lipoxygenase inhibitor is between 5 to 8 days.

7. A process as claimed in claim 2 wherein the medium is inoculated with 5 to 15% of the inoculum.

8. A process as claimed in claim 2 wherein the fermented medium is extracted with water immiscible solvents such as chloroform and ethylacetate.

9. A process as claimed in claim 2 wherein the solvents for column chromatography are hexane and chloroform.

10. An in vitro method of using 8-methyl 13-phenyl trideca 4,6,8,10,12 pentene-3-one as a human platelet aggregation inhibitor and as inhibitor of soybean lipoxygenase.

## Patentansprüche

1. Verfahren zur Herstellung von 8-Methyl-13-phenyl-trideca-4,6,8,10,12-penten-3-on, das sich als Blutplättchenaggregationshemmer und Sojabohnen-Lipoxygenasehemmer eignet, wobei dieses Verfahren die folgenden Schritte umfaßt:
i) Entwickeln eines Mediums, das Kartoffel-Dextrose-Bouillon umfaßt und einen pH-Wert von 5-5,5 aufweist,
ii) Inokulieren des Mediums aus Schritt (i) mit Pilzen der Gattung Aspergillus sowie 5- bis 8-tägiges Inkubieren der Kultur bei 28° bis 37°C,
iii) Versetzen der Kultur mit Essigester und 1-bis 3-stündiges Inkubieren bei Raumtemperatur,
iv) Abfiltrieren des Lösungsmittels von der Fermentationsbrühe,
v) Entfernen jeglichen Wassers aus der Kultur und Heruntertrocknen auf einen Rohextrakt,
vi) Durchführen einer Säulenchromatographie mit dem Rohextrakt und Eluieren der Verbindung 8-Methyl-13-phenyltrideca-4,6,8,10,12-penten-3-on von der orangefarbenen Fraktion in Form von roten Kristallen sowie
vii) Aufreinigen der Verbindung nach traditionellen Verfahren.

2. Verfahren zur Herstellung von 8-Methyl-13-phenyl-trideca-4,6,8,10,12-penten-3-on, das sich als Human-Blutplättchenaggregationshemmer und Sojabohnen-Lipoxygenasehemmer eignet, bei dem man ein Inokulum für die Fermentation **dadurch** entwickelt, daß man die Mycelien oder Sporen von *Aspergillus* von einem 3- bis 5-Tage alten Kartoffel-Dextrose-Schrägagar abschabt, in 500-2000 ml Kartoffel-Dextrose-Bouillonlösung überführt und 24-48 Stunden unter Schütteln bei 100-200 U/min bei 28-37° inkubiert und so die Brühe fermentiert, wobei es sich bei dem Medium, das sowohl für die Entwicklung des Inokulums als auch für die Hauptfermentation verwendet wird, um Kartoffel-Dextrose-Bouillon handelt, die gekochte 15-20 g geschälte zerschnittene Kartoffeln und 1-3 g Glucose pro 100 ml Medium enthält, wobei der EndpH 5,1-5,5 beträgt und man das Medium 10-20 Minuten lang bei 121°C sterilisiert und auf 30°C abkühlen läßt, bevor inokuliert wird, indem man 0,5-2 Liter Inokulum in das Flüssigmedium überführt und 5-8 Tage lang bei 100-200 U/min bei 28-30°C inkubiert und nach dem Ende der Fermentation das fermentierte Medium mit 1-5 Litern Lösungsmittel wie Essigester versetzt und 1-3 Stunden lang bei Raumtemperatur inkubiert, wonach man die Kulturbrühe durch ein Whatman Nr. 1 Papierfilter filtriert und das Lösungsmittel mit einem Scheidetrichter abtrennt, mit 10-50 g wasserfreiem Natriumsulfat versetzt, um alle Wasserreste zu entfernen, und das Lösungsmittel im Vakuum destilliert, wodurch man 0,5-2 g Rohextrakt erhält, der mittels Säulenchromatographie aufgereinigt wird, wobei man eine Kombination von polaren und unpolaren Lösungsmitteln verwendet und den Rohextrakt auf 10-50 g Silicagelsäule (100-200 Mesh) in Hexan aufträgt, wobei die Säule ein Bettvolumen von 20-60 ml aufweist, wonach die Säule erst mit 1-2 Volumina Hexan gewaschen wird, wonach der Hemmstoff mit 1-2 Volumina Hexan:Chloroform (1:1) in Form einer orangefarbigen Fraktion eluiert wird, die im Vakuum destilliert wird, um das Lösungsmittel zu entfernen, wodurch man zu 100-200 mg dieses aufgereinigten Hemmstoffs gelangt, der mit 1-10 ml Hexan versetzt und mindestens 2-3 Tage lang bei 1-10°C aufbewahrt wird, wodurch man zu 1-5 mg aufgereinigtem Hemmstoff in Form von roten Kristallen gelangt, wobei dieser Hemmstoff zum Schluß eine ic₅₀-Aktivität von 234 µM und 95,6 µM gegen Human-Blutplättchenaggregation bzw. Lipoxygenasehemmung aufweist.

3. Verfahren nach Anspruch 2, wobei die Pilze aus der Reihe *Aspergillus flavus, Aspergillus japonicus, Aspergillus niger* und *Aspergillus awamori* stammen.

4. Verfahren nach Anspruch 2, wobei das Medium in Schritt (i) Kartoffel und Glucose umfaßt.

5. Verfahren nach Anspruch 2, wobei die Aspergillus 3-5 Tage lang in einem Medium, das Kartoffel, Dextrose und Agar enthält, unter Schütteln bei 100-200 U/min gezüchtet werden.

6. Verfahren nach Anspruch 2, wobei die Fermentationszeit für die Produktion des Human-Blutplättchenaggregationshemmers und des Sojabohnen-Lipoxygenasehemmers zwischen 5 und 8 Tagen liegt.

7. Verfahren nach Anspruch 2, wobei das Medium mit 5 bis 15% Inokulum inokuliert wird.

8. Verfahren nach Anspruch 2, wobei das fermentierte Medium mit mit Wasser nicht mischbaren Lösungsmitteln wie Chloroform und Essigester extrahiert wird.

9. Verfahren nach Anspruch 2, wobei es sich bei dem Lösungsmittel für die Säulenchromatographie um Hexan und Chloroform handelt.

10. In-vitro-Verfahren für die Verwendung von 8-Methyl-13-phenyltrideca-4,6,8,10,12-penten-3-on als Human-Blutplättchenaggregationshemmer und als Hemmer der Sojabohnen-Lipoxygenase.

## Revendications

1. Procédé de production de 8-méthyl-13-phényltridéca-4,6,8,10,12-pentén-3-one, utile comme inhibiteur de l'agrégation des plaquettes et inhibiteur de la lipoxygénase de soja, ledit procédé comprenant les étapes :
i) de développement d'un milieu comprenant un bouillon de dextrose de pomme de terre et ayant un pH de 5-5,5,
ii) d'inoculation du milieu de l'étape (i) par des champignons de l'espèce Aspergillus et d'incubation de la culture à une température de 28° à 37° pendant 5 à 8 jours,
iii) d'addition d'acétate d'éthyle à la culture et l'incubation de celle-ci à température ambiante pendant 1-3 heures,
iv) de séparation du solvant du bouillon de fermentation par filtration,
v) d'élimination de toute trace d'eau de la culture, et de séchage de celle-ci pour obtenir un extrait brut,
vi) de soumission de l'extrait brut à une chromatographie sur colonne et d'élution du composé, la 8-méthyl-13-phényltridéca-4,6,8,10,12-pentén-3-one, sous forme de cristaux rouges à partir de la fraction de couleur orange, et
vii) de purification du composé par des méthodes classiques.

2. Procédé de production de 8-méthyl-13-phényltridéca-4,6,8,10,12-pentén-3-one, utile comme inhibiteur de l'agrégation des plaquettes humaines et inhibiteur de la lipoxygénase de soja, comprenant le développement d'un inoculum pour la fermentation en raclant les mycélia ou les spores d'*Aspergillus* à partir d'une gélose inclinée de dextrose de pomme de terre âgée de 3-5 jours et en les transférant dans une solution de bouillon de dextrose de pomme de terre de 500-2000 ml et en incubant à 28-37° pendant 24-48 heures sous agitation à 100-200 rpm, et en fermentant ainsi le bouillon, **caractérisé en ce que** le milieu utilisé tant pour le développement de l'inoculum que pour la fermentation principale est un bouillon de dextrose de pomme de terre comprenant 15-20 g de pommes de terre découpées, pelées et bouillies et 1-3 g de glucose pour 100 ml de milieu, le pH final étant de 5,1-5,5 et le milieu étant stérilisé à 121°C pendant 10-20 minutes et laissé à refroidir jusqu'à 30°C avant l'inoculation, dans lequel 0,5-2 litres d'inoculum sont transférés dans le milieu liquide et incubés à 28-30°C pendant 5-8 jours à 100-200 rpm, et à la fin de la fermentation, 1-5 litres de solvant tel que l'acétate d'éthyle sont ajoutés au milieu fermenté et incubés à température ambiante pendant 1-3 heures, ensuite le bouillon fermenté est filtré sur du papier filtre Whatman n°1 et le solvant est séparé à l'aide d'une ampoule à décanter, 10-50 g de sulfate de sodium anhydre étant ajoutés pour éliminer toute trace d'eau résiduelle, et le solvant étant distillé sous vide pour donner un extrait brut de 0,5-2 g qui est purifié par chromatographie sur colonne en employant une association de solvants polaires et non polaires, dans laquelle l'extrait brut est déposé sur 10-50 g d'une colonne de gel de silice (100-200 mesh) dans l'hexane, où la colonne a un volume de lit de 20-60 ml, et ensuite la colonne est d'abord lavée par 1-2 volumes d'hexane puis l'inhibiteur est élué en utilisant 1-2 volumes d'hexane:chloroforme (1:1) sous la forme d'une fraction de couleur orange, qui est distillée sous vide pour éliminer le solvant et obtenir 100-200 mg de cet inhibiteur semi-purifié, auquel on ajoute 1-10 ml d'hexane et on conserve à 1-10°C pendant au moins 2-3 jours pour obtenir 1-5 mg dudit inhibiteur purifié sous la forme de cristaux rouges ayant une activité de CI₅₀ finale de 234 µM et de 95,6 µM d'inhibition de l'agrégation des plaquettes humaines et d'inhibition de la lipoxygénase, respectivement.

3. Procédé selon la revendication 2, **caractérisé en ce que** les champignons sont choisis parmi *Aspergillus flavus, Aspergillus japonicus, Aspergillus niger* et *Aspergillus awamori.*

4. Procédé selon la revendication 2, **caractérisé en ce que** le milieu dans l'étape (i) comprend de la pomme de terre et du glucose.

5. Procédé selon la revendication 2, **caractérisé en ce que** les Aspergillus sont cultivés pendant 3-5 jours dans un milieu contenant de la pomme de terre, du dextrose et de la gélose sous agitation à 100-200 rpm.

6. Procédé selon la revendication 2, **caractérisé en ce que** le temps de fermentation pour la production de l'inhibiteur de l'agrégation des plaquettes humaines et de l'inhibiteur de la lipoxygénase de soja va de 5 à 8 jours.

7. Procédé selon la revendication 2, **caractérisé en ce que** le milieu est inoculé par 5 à 15% de l'inoculum.

8. Procédé selon la revendication 2, **caractérisé en ce que** le milieu fermenté est extrait par des solvants non miscibles avec l'eau tels que le chloroforme et l'acétate d'éthyle.

9. Procédé selon la revendication 2, **caractérisé en ce que** les solvants pour la chromatographie sur colonne sont l'hexane et le chloroforme.

10. Méthode *in vitro* d'utilisation de la 8-méthyl-13-phényltridéca-4,6,8,10,12-pentén-3-one comme inhibiteur de l'agrégation des plaquettes humaines et comme inhibiteur de la lipoxygénase de soja.
